Europäisches Patentamt

European Patent Office

Office européen des brevets

(11) Veröffentlichungsnummer: **0 305 999**
**A1**

## EUROPÄISCHE PATENTANMELDUNG

(21) Anmeldenummer: 88114204.6

(51) Int. Cl.⁴: **A61F 5/01**

(22) Anmeldetag: 31.08.88

(30) Priorität: 01.09.87 DE 3729197

(43) Veröffentlichungstag der Anmeldung:
08.03.89 Patentblatt 89/10

(84) Benannte Vertragsstaaten:
AT BE CH DE ES FR GB IT LI NL SE

(71) Anmelder: **Thanner, Arthur**
**Am Fallenweg 30**
**D-8884 Höchstädt/Donau(DE)**

(72) Erfinder: **Thanner, Arthur**
**Am Fallenweg 30**
**D-8884 Höchstädt/Donau(DE)**

(74) Vertreter: **Schmitz, Hans-Werner, Dipl.-Ing. et al**
**Schoppe . Schmitz . Weber Patentanwälte**
**Ludwig-Ganghofer-Strasse 20**
**D-8022 Grünwald bei München(DE)**

(54) **Fussgelenk-Stützmanschette.**

(57) Die Erfindung betrifft eine Fußgelenk-Stützmanschette (1), die mit Seitenteilen (2, 2') versehen ist,
in denen Aufnahmetaschen (10, 11 und 10', 11')
angeordnet sind. Diese Aufnahmetaschen (10, 11,
10', 11') weisen jeweils eine Einführöffnung (12, 13
und 12', 13') auf, die mit einem öffenbaren Verschluß (14, 14') versehen sind. In die Aufnahmetaschen (10, 11 und 10', 11') können Versteifungsteile
eingeführt werden. Dadurch ist es möglich, optimale
Anpassungen an unterschiedliche Anwendungsfälle
und den Behandlungszeitpunkt durchzuführen.

FIG.1

EP 0 305 999 A1

## Fußgelenk-Stützmanschette

Die Erfindung betrifft eine Fußgelenk-Stützmanschette mit seitlichen Versteifungsteilen in Aufnahmetaschen nach dem Oberbegriff des Anspruchs 1.

Eine derartige Fußgelenk-Stützmanschette ist aus der US-PS 1 084 197 bekannt. Diese Stützmanschette weist Aufnahmetaschen für Versteifungsteile auf, die aus geraden Stäben bestehen. Dementsprechend sind auch die Aufnahmetaschen geradlinig ausgebildet und erstrecken sich über einen Bereich der Seitenteile, der bei angelegter Fußgelenk-Stützmanschette über den Knöchel verläuft. Dies stellt einen ersten Nachteil der gattungsgemäßen Stützmanschette dar, da selbst bei Vorsehen einer Polsterung ein unerwünschtes und unangenehmes Drücken der Versteifungsteile auf den Knöchel nicht in ausreichendem Maße verhindert werden kann.

Des weiteren weist die bekannte Stützmanschette über den gesamten vorderen Längsrand oberhalb der Einführöffnungen eine harte umlaufende Abschlußkante auf, von der aus überdies die Schließlaschen nach innen geklappt sind, so daß es zu einem Abschnüren des Fußes und zu weiteren unerwünschten Druckstellen kommen kann.

Es ist daher Aufgabe der vorliegenden Erfindung, eine Stützmanschette der im Oberbegriff des Anspruchs 1 angegebenen Art zu schaffen, die eine flexible Anpassung einer optimierten therapeutischen Stützwirkung an das jeweilige Krankheitsbild und den Fortschritt des Heilungsprozesses bei gleichzeitigem hohem Tragekomfort ermöglicht, wobei die Fußgelenkmanschette in normalen handelsüblichen Schuhen tragbar sein soll.

Die Lösung dieser Aufgabe erfolgt durch die Merkmale des Anspruches 1.

Dadurch wird zunächst eine erhebliche Verbesserung der Anpassung an die anatomischen Gegebenheiten des Fußgelenkes erreicht, da die Knöchel bei angelegter Stützmanschette nicht mit den Versteifungsteilen in Berührung kommen, so daß unangenehme Druckstellen sicher vermieden werden können.

Dabei werden gleichzeitig ein unerwünschtes Abschnüren des Fußes und schmerzhafte Druckstellen verhindert, obwohl durch die spezifische Anordnung der Aufnahmetaschen und der in ihnen angeordneten Versteifungsteilen eine hohe seitliche Stützwirkung erreicht wird.

Also ist es mit der erfindungsgemäßen Stützmanschette auf besonders einfache und vorteilhafte Weise möglich, die geforderte seitliche Stützwirkung zu erreichen, ohne die Beweglichkeit des Fußes in Richtungen einzuschränken, in denen keine Stützwirkung erforderlich ist. Dies macht die erfindungsgemäße Stützmanschette für eine Vielzahl von Anwendungsfällen brauchbar, was durch bisher bekannte Stützmanschetten nicht zufriedenstellend erreichbar war.

Mit besonderem Vorteil ist es mit der erfindungsgemäßen Stützmanschette möglich, die Stützwirkung in angelegtem Zustand unter optimaler Anpassung an die zu behandelnde Verletzung einzustellen, wobei zu den weiteren Vorteilen gehört, daß die Stützmanschette Tag und Nacht und in Kombination mit nahezu jeglichem üblichem Schuh getragen werden kann. Des weiteren kann die Stützmanschette bei geeigneter Materialwahl auch dann getragen werden, wenn, wie beispielsweise beim Wassersport, eine Benetzung des Fußes mit Wasser nicht auszuschließen ist.

Des weiteren ist es aus medizinischen Gründen wünschenswert, das Fußgelenk im Endstadium des Heilungsprozesses wieder an Belastungen zu gewöhnen, die denjenigen zumindest nahekommen, die auftreten, wenn keine Stützmanschette getragen wird. Eine derartige sukzessive Verringerung der Stützkraft ist mit der erfindungsgemäßen Stützmanschette in optimierter Art und Weise möglich, da diese bei noch nicht weit fortgeschrittenem Heilungsprozeß mit Versteifungsteilen je nach Verletzungsart unterschiedlich hoher Festigkeit versehen werden kann und bei nahezu wieder gesundem Fußgelenk es sogar möglich ist, sämtliche Versteifungsteile zu entfernen, so daß die Stützwirkung lediglich nur aus der Eigensteifigkeit des Schaftes der Stützmanschette resultiert.

Ferner ergeben sich Vorteile, da die Knöchel nicht mit den Versteifungsteilen in Berührung kommen, was das Vermeiden unangenehmer Druckstellen ermöglicht.

Die Unteransprüche haben vorteilhafte Weiterbildungen der Erfindung zum Inhalt.

Bei der Weiterbildung nach Anspruch 2 ergibt sich der Vorteil einer Vereinfachung der Konstruktion, die darüber hinaus Gewicht einspart und die Dicke der Stützmanschette erheblich herabsetzt.

Bei der Weiterbildung nach Anspruch 3 ergibt sich der Vorteil, daß auch beim Einsetzen von relativ harten Versteifungsteilen das Tragegefühl nicht beeinträchtigt wird. Dies wird ferner durch die vorteilhafte Weiterbildung nach Anspruch 4 verbessert.

Der Tragekomfort wird ferner durch die vorteilhaften Weiterbildungen gemäß den Ansprüchen 5 und 6 erheblich erhöht.

Bei der Weiterbildung nach Anspruch 7 ergibt sich der Vorteil, daß ein unerwünschtes Abschnüren des Fußes verhindert werden kann und darüber hinaus die Beweglichkeit des Fußes nach unten bzw. oben relativ zum Unterschenkel trotz hoher seitlicher Stützwirkung nicht eingeschränkt wird.

Die Ausbildung der Verschlußeinrichtung als Schnürverschluß, der Ösen oder Chili (Halbrundösen gemäß einem im Schuhmacherhandwerk gebräuchlichen Ausdruck) aufweisen kann, stellt eine besonders günstige Ausführung dar, während ein Klettverschluß vorteilhaft ist, da die Manschette auch mit einer Hand problemlos geschlossen und geöffnet werden kann.

Die Weiterbildung nach Anspruch 9 ist insofern von besonderem Vorteil, als das Verhindern eines Abschnüren des Fußes weiterhin unterstützt wird und darüber hinaus das Gewicht und die Größe der Stützmanschette noch weiter herabgesetzt werden können.

Die vorteilhafte Weiterbildung nach Anspruch 10 stellt schließich eine anatomisch besonders günstige Bemessung dar, die vor allem in Kombination mit der vorteilhaften Weiterbildung nach Anspruch 7 besonders günstige Verhältnisse für die Beweglichkeit des Fußes bei hoher Stützwirkung und bei gleichzeitiger Verhinderung von unerwünschten Abschnürungen schafft.

Nachfolgend wird ein Ausführungsbeispiel vorliegender Erfindung anhand der Zeichnung näher erläutert.

Es zeigt:

Fig. 1 eine Seitenansicht einer erfindungsgemäßen Fußgelenk-Stützmanschette; und

Fig. 2 eine Seitenansicht eines Versteifungsteiles.

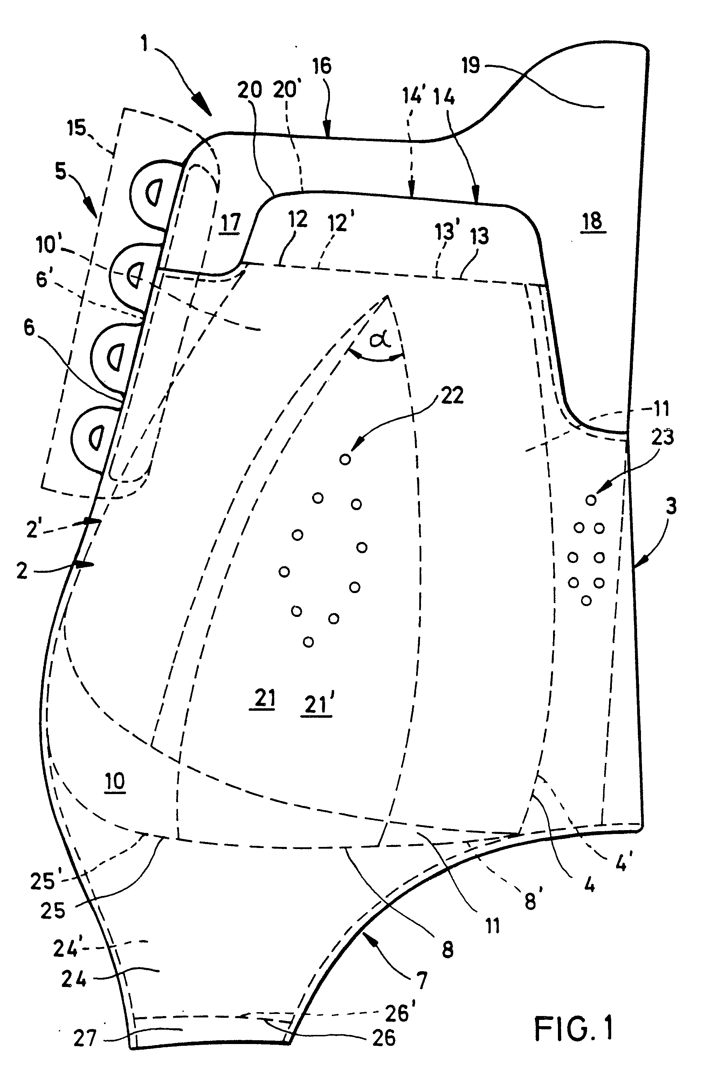

Gemäß Fig. 1 ist eine erfindungsgemäße Fußgelenk-Stützmanschette 1 in zusammengedrücktem Zustand dargestellt, so daß ihre beiden Seitenteile 2, 2' aufeinanderliegen, und mithin lediglich das obere Seitenteil 2 sichtbar ist. Da die Zeichenebene die Teilungsebene der Stützmanschette 1 darstellt, und die auf beiden Seiten dieser Teilungsebene angeordneten Teile bis auf wenige Ausnahmen identisch ausgebildet sind, werden im folgenden die in der Zeichnung nicht sichtbaren Teile jeweils mit den gleichen, jedoch indizierten Bezugsziffern bezeichnet.

Die zwei Seitenteile 2, 2' sind an jeweils einem ihrer Längsränder 4, 4' mittels einem rückseitigen Verbindungsteil 3 miteinander verbunden, von dem in Fig. 1 aufgrund der gewählten Darstellungen nur eine Hälfte sichtbar ist. Das Verbindungsteil 3 selbst ist einstückig ausgebildet und beispielsweise mit einer Steppnaht an den Längsrändern 4, 4' der Seitenteile 2, 2' befestigt. Es ist jedoch auch möglich, die Seitenteile 2, 2' und das Verbindungsteil 3 einstückig auszubilden.

Am anderen gegenüberliegenden Längsrand 6 bzw. 6' des Seitenteiles 2 bzw. 2' ist eine Verschlußeinrichtung 5 vorgesehen. Diese ist bei der in Fig. 1 dargestellten Ausführungsform als Schnürverschluß ausgebildet. Dieser weist im Beispielsfalle acht Chiliösen auf, von denen jeweils vier an jedem der Längsränder 6, 6' angeordnet sind. Durch die Chiliösen kann ein in Fig. 1 nicht dargestelltes Schnurband hindurchgeführt werden. Des weiteren weist der Verschluß eine in Fig. 1 strichliert angedeutete gepolsterte Zunge 15 auf.

Die beiden Seitenteile 2, 2' sind ferner an ihren jeweiligen unteren Rändern 8, 8' mittels eines unteren Verbindungsteiles 7 miteinander verbunden, das im folgenden näher beschrieben werden wird.

Ferner ist aus Fig. 1 ersichtlich, daß bei der dargestellten Ausführungsform jedes Seitenteil 2 bzw. 2' zwei Aufnahmetaschen 10 und 11 bzw. 10' und 11' aufweist. Diese sind an ihrem in der Zeichnung oberen Rand mit Einführöffnungen 12 und 13 bzw 12' und 13' versehen, die wiederum einen öffenbaren Verschluß 14 bzw. 14' aufweisen. Hierbei wird aus Fig. 1 deutlich, daß die bei getragener Manschette 1 außen angeordnete Aufnahmetasche 10 ein beträchliches Stück länger ist, als die im Seitenteil 2' angeordnete entsprechende vordere Aufnahmetasche 10'. Dies trifft ebenso auf die hinteren Aufnahmetaschen 11 und 11' zu, obwohl hier der Längenunterschied etwas geringer ist.

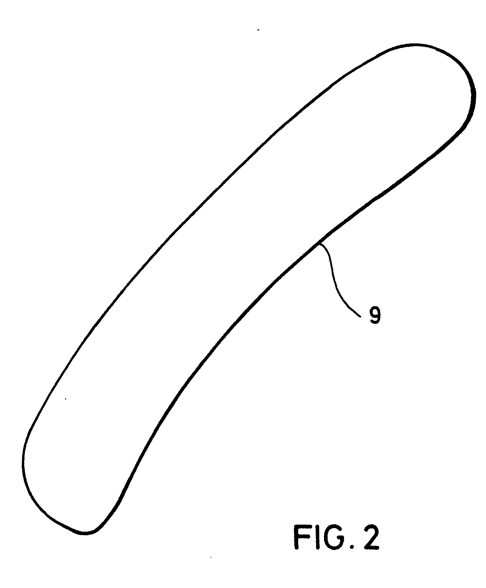

In die Aufnahmetaschen 10, 11, 10', 11' können an deren Größe jeweils angepaßte Versteifungsteile 9 eingeführt werden, von denen eines schematisch leicht vereinfacht in Fig. 2 dargestellt ist. Aus dieser Darstellung geht hervor, daß das Versteifungsteil 9 vorzugsweise stabförmig ausgebildet ist und eine leichte Krümmung sowie abgerundete Enden aufweist. Das Versteifungsteil 9 kann aus unterschiedlichen Materialien unterschiedlicher Härte hergestellt werden, wobei insbesondere thermoplastisch verformbare Materialien mit besonderem Vorzug verwendet werden können. Aus der unterschiedlichen Form und Länge der Aufnahmetaschen 10, 10' und 11, 11' und der entsprechend unterschiedlicher Ausbildung der Versteifungsteile 9 resultiert der Vorteil nochmals verbesserter Anpassungsmöglichkeiten an unterschiedliche Fußformen und daraus resultierende Vermeidung von Druckstellen.

Obwohl in Fig. 1 eine Ausführungsform der Stützmanschette 1 mit insgesamt vier Aufnahmetaschen 10, 11, 10', 11' dargestellt ist, ist es auch möglich, weniger Aufnahmetaschen vorzusehen. Desweiteren ist es möglich, zur Anpassung an unterschiedliche Behandlungszustände und/oder Anwendungsfälle eine unterschiedliche Anzahl von Versteifungsteilen 9 in die vorhandenen Aufnahmetaschen einzuführen. Es können also sowohl alle Aufnahmetaschen 10, 11, 10', 11' mit jeweils einem Versteifungsteil 9 versehen werden, wie es auch im anderen extremen Fall möglich ist, keine der Aufnahmetaschen 10, 11, 10', 11' mit einem Versteifungsteil 9 zu versehen, so daß die Stützwirkung dann nur noch durch die Eigensteifigkeit des von den Seitenteilen 2, 2' und dem Verbindungsteil 3 gebildeten Schaftes erzeugt wird.

Bei der in Fig. 1 dargestellten besonders bevorzugten Ausführungsform der Stützmanschette 1 ist der Schaft ferner mit einem im wesentlichen U-förmigen Verlängerungsteil 16 versehen, dessen beide Schenkel 17 und 18 unterschiedlich lang sind, wobei der Schenkel 18 zudem ein weit heraufgezogenes Verlängerungsteil 19 aufweist.

Die zuvor beschriebenen Seitenteile 2, 2', das Verbindungsteil 3, das Verbindungsteil 7 sowie das Teil 16 bestehen aus Ober- und Futtermaterial, das vorzugsweise zusammengesteppt ist, wobei zwischen dem Ober- und dem Futtermaterial eine Polsterung eingearbeitet ist, um Druckstellen zu vermeiden. Hierbei ist vorzugsweise das Obermaterial des Teiles 16 welcher als das Obermaterial der übrigen zuvor genannten Teile, um eine erhöhte Flexibilität des Teiles 16 zu erreichen. Ferner können das Futtermaterial von Teil 3 und 16 einstückig sein. Allgemein kann als Material Leder oder lederähnliches abwaschbares Synthetikmaterial verwendet werden.

Wie aus Fig. 1 ferner ersichtlich ist, erstrecken sich die Aufnahmetaschen 10, 11, 10', 11' entlang der Längsränder 4, 4' bzw. 6, 6' und laufen in einem spitzen Winkel α zusammen, wobei die Einführöffnungen 12, 12' und 13, 13' im Bereich dieses Winkels α angeordnet sind. Daraus ergibt sich der Vorteil, daß für jeweils zwei Taschen 10, 11 bzw. 10', 11' jeweils nur ein Verschluß 14 bzw. 14' vorgesehen werden muß, mittels dessen beide Taschen 10, 11 bzw. 10', 11' geöffnet bzw. geschlossen werden können. Bei der in Fig. 1 dargestellten Ausführungsform ist der Verschluß 14 bzw. 14' als Klettverschluß ausgebildet. Dieser weist jeweils eine schwenkbar angeordnete Lasche 20 bzw. 20' auf, deren Innenseite mit einem Klettbelag versehen ist, der mit einem auf der Außenseite des Teils 16 angeordneten Klettbelag zusammenwirkt.

Als Alternative kann der Verschluß 14, 14' jedoch auch als Reißverschluß ausgebildet sein, was den Vorteil einer weiteren Herabsetzung der Dicke der Manschette 1 ergibt.

Wie zuvor bereits erwähnt wurde, ist zwischen Ober- und Futtermaterial der Stützmanschette 1 eine Polsterung eingearbeitet, die im Bereich der Aufnahmetaschen dicker ausgebildet ist als in dem zwischen den Aufnahmetaschen befindlichen Bereich, der mit dem Bezugszeichen 21 gekennzeichnet ist. Dies ergibt den besonderen Vorteil einer verbesserten Knöchelentlastung, wobei der Tragekomfort weiterhin durch das Vorsehen einer Mehrzahl von Belüftungsöffnungen 22 im Bereich 21 erhöht wird. Das rückseitige Verbindungsteil 3 kann ebenfalls mit derartigen Belüftungsöffnungen versehen sein, die mit dem Bezugszeichen 23 bezeichnet sind.

Wie Fig. 1 ferner verdeutlicht, ist die Verschlußeinrichtung 5 nur über den oberen Teilbereich der Längsränder 6 bzw. 6' angeordnet, so daß der auf den Spann des Fußes zulaufende Bereich der Stützmanschette 1 selbst bei verschlossener Verschlußeinrichtung nicht zusammengehalten wird. Daraus ergibt sich insbesondere der Vorteil, daß eine Einschnürung des Fußes wirksam verhindert wird, und darüber hinaus wird die Beweglichkeit des Fußes bei Aufrechterhaltung einer hohen Stützwirkung verbessert. Darüber hinaus ergibt sich der Vorteil, daß die erfindungsgemäße Stützmanschette 1 in dem Bereich des Fußes, der bei angezogenem Schuh von dessen Teilen bedeckt wird, praktisch keinen Platzbedarf hat, so daß alle Arten von Schuhen bei angelegter Manschette getragen werden können.

Diese vorteilhaften Wirkungen werden bei der in Fig. 1 dargestellten Ausführungsform der Stützmanschette 1 noch dadurch erhöht, daß das untere Verbindungsteil 7 zwei annähernd trapezförmige Wandteile 24 und 24' aufweist, die über ihre längeren Seiten 25 bzw. 25' jeweils mit den unteren Rändern 8 bzw. 8' verbunden sind. Die kürzeren Seiten 26 bzw. 26' hingegen sind miteinander mittels eines Verbindungssteges 27 verbunden. Dieser Steg ist im wesentlichen rechteckförmig ausgebildet und weist vorzugsweise eine Länge auf, die ungefähr einem Drittel der Länge des unteren Randes 8 bzw. 8' entspricht. Daraus ergibt sich die im einzelnen aus Fig. 1 ersichtliche Ausbildung des Bereiches der Stützmanschette 1, die bei angelegter Manschette 1 die Fußsohle umfaßt. In Zusammenwirkung mit der Verschlußeinrichtung 5 ergeben sich daraus besonders vorteilhafte Verhältnisse, die gleichzeitig das Aufrechterhalten einer hohen Stützwirkung im Zusammenhang mit großer Fußbeweglichkeit und Vermeidung der Gefahr von Einschnürungen ergeben. Alles in allem wird mit der erfindungsgemäßen Fußgelenk-Stützmanschette 1 eine Stützeinrichtung geschaffen, die insbesondere bei Bänderschwächen und zur Vorbeugung oder Nachbehandlung nach Operationen verwendet werden kann. Darüber hinaus kann sie vorteilhafterweise zu praktisch jeglicher Art von Schuh getragen werden und ist insbesondere für Einsatzfälle geeignet, bei denen vor allem das Sprunggelenk besonderen Belastungen ausgesetzt ist.

Wie Fig. 1 bildlich verdeutlicht, sind die Versteifungsteile 9 hoch genug über das Sprunggelenk gezogen, um einen optimalen seitlichen Halt zu gewähren, und durch die zuvor beschriebene Ausbildung der Verschlußeinrichtung 5 im Zusammenhang mit dem Verbindungsteil 7, das die Ferse freiläßt, wird hohe Beweglichkeit garantiert.

Als Abwandlungsmöglichkeit kann die Stützmanschette 1 als Sprunggelenk- und Mittelfußbandage ausgebildet sein, die im wesentlichen den gleichen Aufbau wie die zuvor beschriebene Manschette hat, jedoch auch mittelfußumfassend geschnürt und mit seitlichen Bewegungseinschnitten in der Beuge versehen ist. Dadurch ist die Manschette 1 insbesondere geeignet bei Bandverletzungen, nach der Gipsabnahme und zur Entlastung und Ruhigstellung des Sprunggelenks.

Ein weiterer Vorteil der erfindungsgemäßen Stützmanschette 1 insbesondere gegenüber Schuhen ist, daß bei nur einem verletzten Fuß auch nur eine Manschette gekauft und getragen werden muß und daß darüber hinaus ein Tragen auch während der Nacht ohne Behinderungen möglich ist.

Darüber hinaus ist es möglich, daß ohne weiteres auch Schuheinlagen zusätzich zur Stützmanschette 1 getragen werden können, da diese insbesondere im Sohlenbereich relativ schmal und klein ausgebildet ist, so daß keine übermäßigen Behinderungen selbst bei angezogenem Schuh zu befürchten sind.

**Ansprüche**

1. Fußgelenk-Stützmanschette (1)
mit zwei Seitenteilen (2, 2'),
mit einem rückseitigen Verbindungsteil (3), das mit den Seitenteilen (2, 2') an deren hinteren Längsrändern (4, 4') verbunden ist,
mit einer öffenbaren Verschlußeinrichtung (5), die an den gegenüberliegenden vorderen Längsrändern (6, 6') der Seitenteile (2, 2') angeordnet ist,
mit einem unteren Verbindungsteil (7), das mit den Seitenteilen (2, 2') an deren jeweiligem unteren Rand (8, 8') verbunden ist, und
mit stabförmigen Versteifungsteilen (9), die in jeweiligen Aufnahmetaschen (10, 11, 10', 11') eines jeden Seitenteiles (2, 2') angeordnet sind,
wobei die Aufnahmetaschen (10, 11, 10', 11') sich entlang der Längsränder (4, 4', 6, 6') erstrecken und jeweils eine Einführöffnung (12, 13, 12', 13') aufweisen, die mit einem öffenbaren Verschluß (14, 14') versehen sind, dadurch gekennzeichnet,
daß die Aufnahmetaschen (10, 11, 10', 11') in spitzem Winkel (α) zusammenlaufen, wobei die Einführöffnungen (12, 13, 12', 13') im Bereich des Scheitelpunktes des Winkels (α) angeordnet sind,
wobei die außen angebrachten Aufnahmetaschen (10, 11) beträchtlich länger sind, als die an der Fußinnenseite angebrachten Aufnahmetaschen (10', 11'),
daß die entfernbaren Verstärkungsteile (7) eine leichte Krümmung aufweisen, und von unterschiedlicher Härte, Form und Längsausdehnung sein können, und
daß der von den Seitenteilen (2, 2') und dem Verbindungsteil (3) gebildete Schaft mit einem gepolsterten U-förmigen Verlängerungsteil (16) versehen ist, dessen Obermaterial weicher ist und eine Flexibilität aufweist, die größer ist, als diejenige des Schaftes.

2. Stützmanschette nach Anspruch 1, dadurch gekennzeichnet, daß jeweils nur ein Verschluß (14, 14') für jeweils zwei Aufnahmetaschen (10, 11, 10', 11') vorgesehen ist.

3. Stützmanschette nach Anspruch 1 oder 2, dadurch gekennzeichnet, daß die Aufnahmetaschen (10, 11, 10', 11') an den Flächen der Seitenteile (2, 2'), die bei angelegter Stützmanschette (1) am Fuß anliegen, mit einer Polsterschicht versehen ist.

4. Stützmanschette nach einem der Ansprüche 1 bis 3, dadurch gekennzeichnet, daß der zwischen den Aufnahmetaschen (10, 11, 10', 11') angeordnete, am Fuß anlegbare Seitenflächenbereich (21, 21') mit einer Polsterschicht versehen ist, die dünner ist als die Polsterschicht der Aufnahmetaschen (10, 11, 10', 11').

5. Stützmanschette nach Anspruch 4, dadurch gekennzeichnet, daß der Seitenflächenbereich (21, 21') mit Belüftungsöffnungen (22) versehen ist, die bei angelegter Manschette (1) im Bereich des Knöchels angeordnet sind.

6. Stützmanschette nach einem der Ansprüche 1 bis 5, dadurch gekennzeichnet, daß das rückseitige Verbindungsteil (3) mit Belüftungsöffnungen (23) versehen ist.

7. Stützmanschette nach einem der Ansprüche 1 bis 6, dadurch gekennzeichnet, daß sich die Verschlußeinrichtung (5) nur über einen Teilbereich der vorderen Längsränder (6, 6'), ausgehend von deren im getragenen Zustand oberen Ende erstreckt.

8. Stützmanschette nach einem der Ansprüche 1 bis 7, dadurch gekennzeichnet, daß die Verschlußeinrichtung (5) als Schnürverschluß oder Klettverschluß ausgebildet ist.

9. Stützmanschette nach einem der Ansprüche 1 bis 8, dadurch gekennzeichnet, daß das untere Verbindungsteil (7) zwei annähernd trapezförmige Wandteile (24, 24') aufweist, die über ihre längeren Seiten (25, 25') jeweils mit den unteren Rändern (8, 8') verbunden sind, während die kürzeren Seiten (26, 26') mittels eines im wesentlichen rechteckförmigen Verbindungssteges (27) miteinander verbunden sind.

10. Stützmanschette nach Anspruch 9, dadurch gekennzeichnet, daß die Länge des Verbindungssteges (27) etwa 30 % der Länge des unteren Randes (8, 8') entspricht.

11. Stützmanschette nach einem der Ansprüche 1 bis 10, dadurch gekennzeichnet, daß der Verschluß (14, 14') als Klettverschluß oder Reißverschluß ausgebildet ist.

12. Stützmanschette nach einem der Ansprüche 1 bis 11, dadurch gekennzeichnet, daß die Versteifungsteile (9) aus thermoplastisch verformbarem Material bestehen.

EP 0 305 999 A1

FIG.1

9

FIG. 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | US-A-4 237 874 (NELSON)<br>* Figuren 1, 3, 4 *<br>--- | 1 | A 61 F 5/01 |
| A | US-A-4 527 556 (NELSON)<br>* Figur 4; spalte 5, Zeilen 22-33 *<br>--- | 1 | |
| A | US-A-4 280 488 (POLSKY et al.)<br>* Figuren 2-4; Spalte 3, Zeilen 28-34 *<br>--- | 1 | |
| A | US-A-4 187 844 (CAPRIO)<br>* Figur 1; Anspruch 1 *<br>--- | 1 | |
| D,A | US-A-1 084 197 (COLLINS)<br>* Figur *<br>--- | 1 | |
| P,X | DE-U-8 711 841 (THANNER)<br>* ganzes Dokument *<br>----- | 1-12 | |

RECHERCHIERTE
SACHGEBIETE (Int. Cl.4)

A 61 F 5/00
A 61 F 13/00

Der vorliegende Recherchenbericht wurde für alle Patentansprüche erstellt

| Recherchenort | Abschlußdatum der Recherche | Prüfer |
|---|---|---|
| BERLIN | 01-12-1988 | KANAL P K |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus andern Gründen angeführtes Dokument
.................................................................
& : Mitglied der gleichen Patentfamilie, übereinstimmendes
    Dokument

EPO FORM 1503 03.82 (P0403)